# EUROPEAN PATENT SPECIFICATION

(11) **EP 3 820 399 B1**
(45) Date of publication and mention of the grant of the patent: **06.03.2024**
(21) Application number: 19833137.3
(22) Date of filing: 12.07.2019
(51) Int. Cl.: A61C 7/06, A61B 17/60, A61B 17/66, A61C 7/10, A61F 13/12, A62B 18/08

(54) **CANTILEVER PROTRACTION DEVICE**
AUSLEGERPROTRAKTIONSVORRICHTUNG
DISPOSITIF DE PROTRACTION EN PORTE-À-FAUX

(30) Priority: 13.07.2018 US 201862697743 P; 12.06.2019 US 201962860630 P
(43) Date of publication of application: 19.05.2021
(73) Proprietor: Craniofacial Technologies Inc., Bell Canyon, California 91307 (US); Kaveh, Cameron, Bell Canyon, CA 91307 (US); Yuschak, Thomas, D., Bell Canyon, CA 91307 (US); Kaveh, Kevin, Bell Canyon, CA 91307 (US)
(72) Inventor: KAVEH, Cameron, Bell Canyon, CA 91307 (US); YUSCHAK, Thomas, D., Bell Canyon, CA 91307 (US); KAVEH, Kevin, Bell Canyon, CA 91307 (US)
(74) Representative: Sach, Greg Robert
(86) International application number: PCT/US2019/041667
(87) International publication number: WO 2020/014648

(56) References cited:
- KR-U- 20110 011 225
- US-A- 5 890 891
- US-A1- 2007 287 900
- US-A1- 2012 247 490
- US-A1- 2018 028 282
- US-A1- 2018 028 282
- US-B1- 6 213 765
- US-B1- 6 213 765

## Description

### BACKGROUND

A large percentage of people are maxillary deficient relative to their genetic potential for maxillary development. In order to achieve this full maxillary development potential, protractionary forces must be applied to the bone. According to Newton's third law of motion, for every action there is an equal and opposite reaction. Therefore, in order to apply this protractionary force on the maxilla, a device must be capable of handling the opposite reaction.

In traditional orthodontics, convenience has taken priority, and many suboptimal physiologic negative anchorage points have been attempted, including the forehead, cheekbones, chin, and neck. However, applying a negative force to sensitive regions of the body for the long periods of time required for protraction is far from ideal and even harmful in many cases. For example, negative force application on the mandible/chin (such as in U.S. Patent No. 8,640,710) is associated with mandibular recession and temporomandibular joint stress generation. Devices using a neck brace immobilize the neck and create large amounts of heat and discomfort in the sensitive neck region. Negative force on the frontal bone or cheekbones can create deformation and recession of the bone structure over time. For example, the maxillary protraction device described in U.S. Patent Publication No. 2018/0028282 places significant loads upon the head and provides only limited head movement due to a body anchor having only a single point of articulation anchored to an immobile location on a patient's abdomen.

There is a need in the art for improved protraction devices. The present invention addresses this need.

### SUMMARY

The object of the present invention is a protraction device, comprising:
a body frame, adapted to be anchored to the chest and abdomen of a patient,
a cantilever support, including
   - a first shaft, coupled to the body frame, and
   - a second shaft, coupled to the first shaft, and
a headpiece coupled to the second shaft and adapted to be attached to the head of the patient, wherein the second shaft extends in front of the face of the patient when the protraction device is worn by said patient, characterized in that the body frame comprises
   - a top rail, and
   - a bottom rail, and
the cantilever support further comprises
   - a top/upper bearing, coupled to the top rail, and
   - a bottom/lower bearing, coupled to the bottom rail, and
the first shaft is coupled to the top/upper bearing and the bottom/lower bearing.

In one embodiment, the frame comprises at least one upper lateral rail and at least one lower lateral rail connected by opposing side rails, the lateral rails being in parallel alignment and having equal curvatures. In one embodiment, the frame is constructed from one or more shaft sections. In one embodiment, at least a portion of the shaft sections are adjustably telescoping.

In one embodiment, the device further comprises at least one strap pad tautly suspended between the opposing side rails, the at least one strap pad comprising padding constructed from a gel or a foam. In one embodiment, the frame comprises a harness having shoulder and waist straps. In one embodiment, the harness and the at least one strap pad are configured to secure the device to a subject such that frame does not physically touch the subject or minimally touches the subject.

In one embodiment, the curvature of the lateral rails is a circular arc having an angle between about 160° and 180°. In one embodiment, the headpiece rail curvature is a circular arc having an angle between about 90° and 140°.

In one embodiment, the device further comprises a linkage arm attached to the headpiece, the linkage arm having a curvature equal to the headpiece rail. In one embodiment, the headpiece further comprises one or more attachments slidably connected to the headpiece rail and lockable to the linkage arm, the one or more attachments selected from a hook attachment, a linear gear bar attachment, and combinations thereof.

In one embodiment, the slidable connections comprise a low friction bearing. In one embodiment, the low friction bearing is constructed from a material selected from polytetrafluoroethylene (PTFE), ultra high molecular weight polyethylene (UHMWPE), and combinations thereof.

In one embodiment, the headpiece is slidable along the headpiece rail in a medial plane and configured to support flexion and extension of a subject's head. In one embodiment, the cantilever support is slidable along the frame along a transverse plane and configured to support rotation of a subject's head. In one embodiment, the attachment between the cantilever support and the headpiece rail comprises a rotatable joint permitting rotation in a coronal plane, the rotatable joint configured to support lateral flexion of a subject's head.

In another aspect, the present description relates to an anchored protraction device, comprising: a rail having a curvature, the rail being attached to an anchor; a head brace slidably connected to the rail; and an elongate linkage arm attached to the head brace.

In one embodiment, the anchor is securable to a gurney, a bed headboard, a floor stand, and combinations thereof. In one embodiment, the device further comprises one or more attachments slidably connected to the rail and lockable to the linkage arm, the one or more attachments selected from a hook attachment, a linear gear bar attachment, and combinations thereof.

In one embodiment, the slidable connections comprise a low friction bearing. In one embodiment, the low friction bearing is constructed from a material selected from polytetrafluoroethylene (PTFE), ultra high molecular weight polyethylene (UHMWPE), and combinations thereof.

In an aspect, a protraction system comprises a therapeutic appliance, a protraction device, and a force applicator. The therapeutic appliance is coupled to a patient. The protraction device includes a body frame and a cantilever support, coupled to the body frame by a roller bearing. The force applicator is removably coupled to the cantilever support and to the therapeutic appliance. The therapeutic appliance is anchored to at least one member selected from the group consisting of the teeth, bone, and soft tissue of the patient.

An aspect, which is not part of the invention, relates to a method of providing maxillary protraction to a patient in need thereof comprising coupling a protraction device to a therapeutic appliance. The therapeutic appliance is coupled to the patient.

In an aspect a headpiece comprises a first strap, a harness, and a second strap. The first strap is configured to encircle the back and sides of the head of a patient. The harness is removably coupled to the first strap by a plurality of fasteners. The second strap is configured to pass under the jaw of the patient and is coupled to the harness. The second strap comprises a mandible fastener. The harness does not obstruct the facial area of the patient.

In an aspect a protraction device comprises an anchor, a cantilever support, a headpiece, and a force applicator. The cantilever support includes a shaft movably coupled to the anchor. The headpiece is adjustably coupled to the shaft. The force applicator is removably coupled to the headpiece and adjustably coupled to the shaft. The anchor is configured to be secured to a supporting object.

In an aspect a protraction system comprises a therapeutic appliance and a trans-oral member. The therapeutic appliance is adapted to be coupled to at least one member selected from the group consisting of the teeth, bone, and soft tissue of a patient. The trans-oral member includes a curved member and an extra-oral vertical member. Each end of the curved member is coupled to opposing ends of the therapeutic appliance. The extra-oral vertical member extends vertically from the plane of the curved member.

### BRIEF DESCRIPTION OF THE DRAWINGS

The following detailed description of embodiments of the invention will be better understood when read in conjunction with the appended drawings. It should be understood, however, that the invention is not limited to the precise arrangements and instrumentalities of the embodiments shown in the drawings.
Figure 1 depicts a side view (left) and a perspective view (right) of an exemplary protraction device.
Figure 2 depicts a perspective view of an exemplary body frame of a protraction device.
Figure 3 depicts a front view (left) and a back view (right) of an exemplary protraction device fitted to a patient with a harness.
Figure 4 depicts a perspective view of an exemplary cantilever support of a protraction device.
Figure 5 depicts a perspective view of an exemplary headpiece of a protraction device.
Figure 6 depicts a perspective view of an exemplary low friction bearing with hook attachment.
Figure 7 depicts a perspective view of an exemplary low friction bearing with rack attachment.
Figure 8 depicts a side view of an exemplary headpiece attached to the head of a patient.
Figure 9 depicts a side view of another exemplary protraction device, but which does not correspond to the present invention.
Figure 10 depicts the results of experiments investigating the effect of normal force on the lateral force needed to move a low friction bearing along an aluminum rail.
Figure 11A illustrates a perspective view of a cantilevered protraction device with a roller bearing.
Figure 11B illustrates a side view of a cantilevered protraction device with a roller bearing.
Figure 11C illustrates an exploded view of a roller bearing.
Figure 12 illustrates a cantilever support with multiple bearings.
Figure 13 illustrates a cantilever support with vertical rollers.
Figure 14A illustrates a cantilever support with multiple bearings having a separation angle of 60°.
Figure 14B illustrates a cantilever support with multiple bearings having a separation angle of 45°.
Figure 14C illustrates a cantilever support with multiple bearings having a separation angle of 30°.
Figure 15 illustrates a cantilever support with support bars.
Figure 16 illustrates a cantilever protraction device with a neck support.
Figure 17 illustrates a cantilever support coupled to a rotational hinge in a configuration which does not correspond to the present invention.
Figure 18 illustrates a cantilever protraction device coupled to a moveable anchorage.
Figure 19A illustrates a headpiece for a cantilever protraction device. Figure 19B illustrates a headpiece for a cantilever protraction device as worn by a user.
Figure 20A illustrates a sectioned view of a force applicator including a constant-force spring.
Figure 20B illustrates a force applicator including a constant-force spring in an extended position.
Figure 20C illustrates a force applicator and a mount for coupling the force applicator to a protraction device.
Figure 21 illustrates an adjustable anchorage device coupled to a protraction wire.
Figure 22A illustrates the finite element analysis of the von Mises stresses of a harness for a cantilever protraction device as measured in pounds per square inch (1 psi=6,895KPa)
Figure 22B illustrates the finite element analysis of the URES displacement of a harness for a cantilever protraction device as measured in inches (1 in=2,54cm).
Figure 23 illustrates a protraction wire coupled to a protraction device and to a skeletal anchorage.
Figure 24 illustrates a partial view of a cantilever protraction device with concealed bearings.
Figure 25A illustrates a side view of an alternative design of a headpiece for a cantilever protraction device as worn by a user.
Figure 25B illustrates a back view of an alternative design of a headpiece for a cantilever protraction device as worn by a user.
Figure 26A illustrates a perspective view of a therapeutic appliance coupled to a trans-oral member.
Figure 26B illustrates a side view of a therapeutic appliance coupled to a trans-oral member.

### DETAILED DESCRIPTION

The present invention provides body anchored protraction devices and off-the-head anchored protraction devices as defined in the claims. The protraction devices direct the negative forces of protraction over a large surface area on the chest and abdomen of a patient. The protraction devices are lightweight and employ a cantilever support rod and ultra-low friction joints to enable low compression on the head with low resistance to head movements.

### Definitions

It is to be understood that the figures and descriptions of the present invention have been simplified to illustrate elements that are relevant for a clear understanding of the present invention, while eliminating, for the purpose of clarity, many other elements typically found in the art. Those of ordinary skill in the art may recognize that other elements are desirable and/or required in implementing the present invention.

Unless defined elsewhere, all technical and scientific terms used herein have the same meaning as commonly understood by one of ordinary skill in the art to which this invention belongs. Although any methods and materials similar or equivalent to those described herein can be used in the practice or testing of the present invention, exemplary methods and materials are described.

As used herein, each of the following terms has the meaning associated with it in this section.

The articles "a" and "an" are used herein to refer to one or to more than one (i.e., to at least one) of the grammatical object of the article. By way of example, "an element" means one element or more than one element.

"About" as used herein when referring to a measurable value such as an amount, a temporal duration, and the like, is meant to encompass variations of ±20%, ±10%, ±5%, ±1%, and ±0.1% from the specified value, as such variations are appropriate.

The term "supporting object" means an object having a rigid surface that is capable of physically supporting an anchored protraction device. Examples of supporting objects include walls, beds, gurneys and stands, such as floor stands.

Throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6, etc., as well as individual numbers within that range, for example, 1, 2, 2.7, 3, 4, 5, 5.3, 6, and any whole and partial increments therebetween. This applies regardless of the breadth of the range.

### Protraction Device

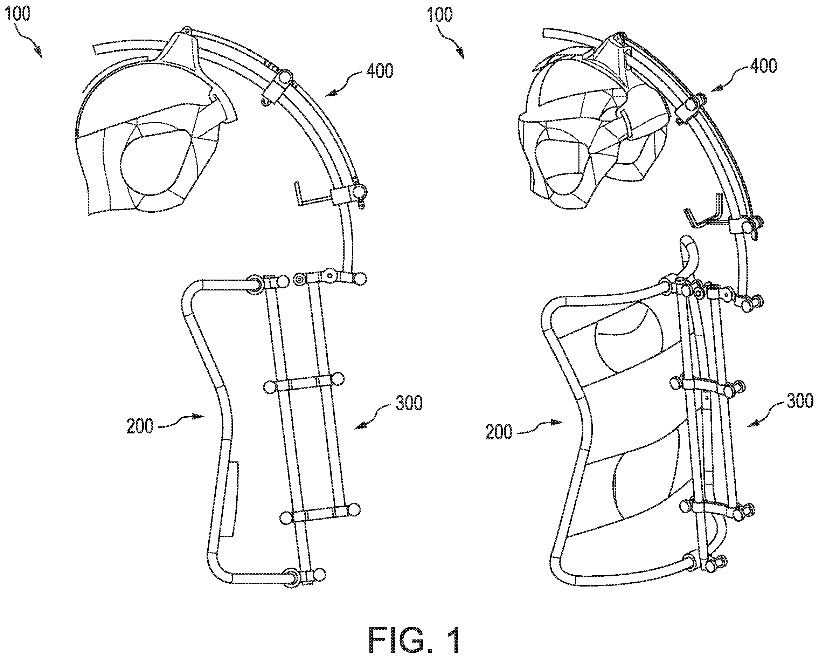

The present invention provides protraction devices that are configured to direct the negative forces of maxillary protraction to the chest and abdomen and away from the sensitive regions of the head and neck of a patient. The devices spread the negative force out over a large surface area to reduce fatigue and discomfort. Wearable devices are lightweight, such as in the range of 1 to 2 Ibs (1lb=0,454Kg) and below, and permit a patient's head to retain substantial freedom of motion, including rotation and nodding. Referring now to Figure 1, an exemplary body anchored protraction device **100** is depicted. Device **100** comprises body frame **200,** cantilever support **300,** and headpiece **400.**

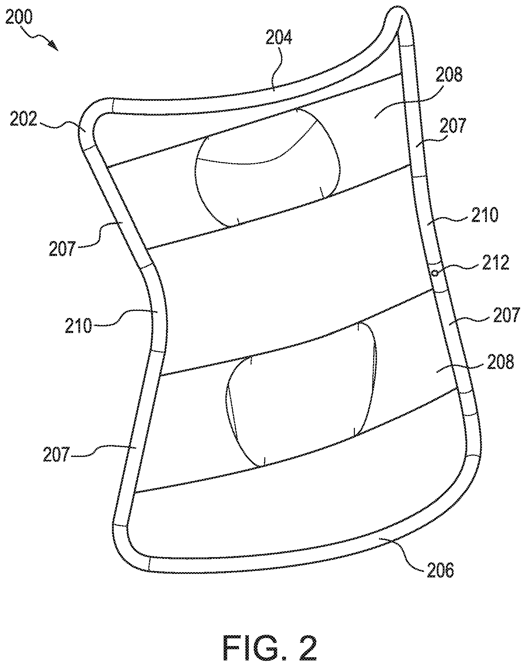

Referring now to Figure 2, body frame **200** is described in detail. Body frame **200** comprises one or more sections of shaft **202** forming a perimeter. Body frame **200** can have any suitable shape, such as a substantially quadrilateral or elliptical shape. Shaft **202** can be hollow or solid and can be constructed from any suitably rigid material, such as aluminum, polycarbonate, or some other lightweight metal, plastic, or composite material. In various embodiments, shaft **202** can have any suitable cross section, including circular, elliptical, square, and rectangular. At a top side and a bottom side, shaft **202** forms upper rail **204** and lower rail **206,** respectively, each connected to opposing side rails **207.** Upper rail **204** and lower rail **206** are aligned in parallel (see Figure 1) and have matching curvatures. In some embodiments, the matching curvatures can be described as a circular arc having an angle between about 160° and 180°. In some embodiments, body frame **200** can comprise one or more additional lateral rails aligned in parallel with upper rail **204** and lower rail **206** and having matching curvatures. The one or more additional lateral rails can be connected to side rails **207,** or to upper rail **204** or lower rail **206,** such as by a short segment of shaft **202.** Side rails **207** can have one or more indents **210** configured to conform to a patient's anatomy, such as the pectoral muscles.

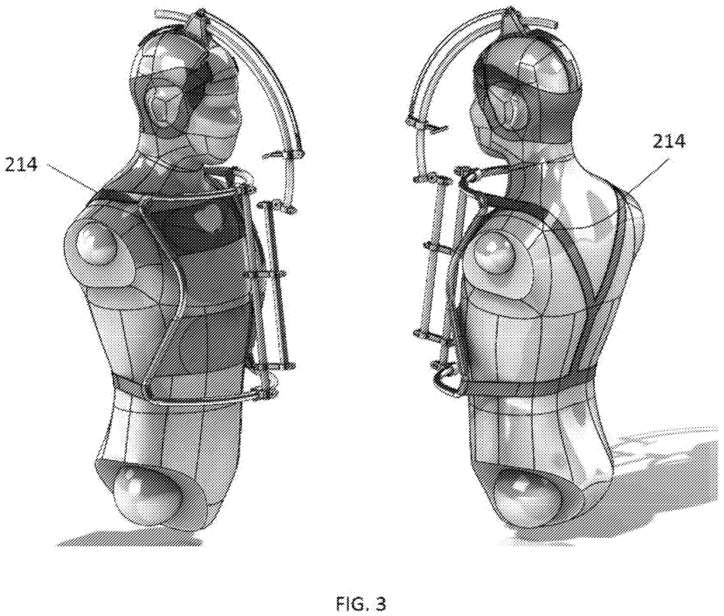

Body frame **200** can be secured to a patient's body using any suitable mechanism. For example, in some embodiments body frame **200** further comprises strap pads **208** suspended between side rails **207.** Strap pads **208** can be constructed from a fabric or polymer mesh and can be fitted with a gel or foam cushion for enhanced comfort and fit. In some embodiments, strap pads **208** are suspended in a taut fashion to support the weight of device **100** and to support lateral forces exerted on device **100.** In another example, body frame **200** can comprise a plurality of rigid or semi-rigid padded feet configured to engage the shoulder and abdomen of a patient and to support and spread out the lateral forces exerted on device **100.** In some embodiments, body frame **200** is size adjustable, wherein sections of shaft **202** are telescoping and lockable by way of one or more locks **212.** Body frame **200** further comprises harness **214** having shoulder and waist straps to secure body frame **200** to a patient (Figure 3). It should be noted that appropriate sizing and fit of body frame **200** secures device **100** to a patient such that frame **200** does not physically contact the patient, or minimally contacts the patient, ensuring that loads are spread out over the patient's body.

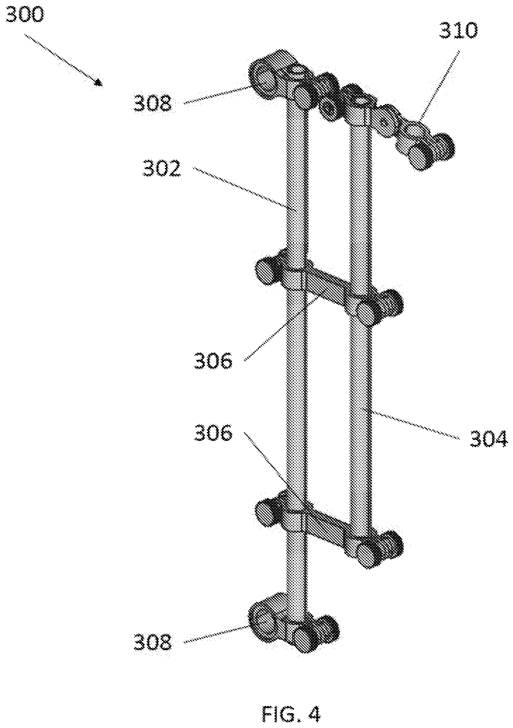

Referring now to Figure 4, cantilever support **300** is described in detail. Cantilever support **300** comprises first shaft **302** and second shaft **304** secured to each other by clamps **306.** Similarly to shaft **202,** first shaft **302** and second shaft **304** can be constructed from any suitably rigid and lightweight material. First shaft **302** comprises low friction bearing **308** at its upper and lower ends, wherein the upper low friction bearing **308** is slidable along upper rail **204** and the lower low friction bearing **308** is slidable along lower rail **206.** In various embodiments, first shaft **302** can comprise additional low friction bearings **308** connectable to and slidable along additional lateral rails on body frame **200,** as described elsewhere herein. Each low friction bearing **308** can be constructed from any durable material having a low coefficient of friction, such as polytetrafluoroethylene (PTFE) and ultra high molecular weight polyethylene (UHMWPE). Second shaft **304** comprises headpiece attachment **310** at its upper end. The positioning of the various components (clamps **306,** low friction bearing **308,** headpiece attachment **310**) are adjustable along their respectively attached first shaft **302** or second shaft **304** by any suitable locking mechanism, such as a ring clamp.

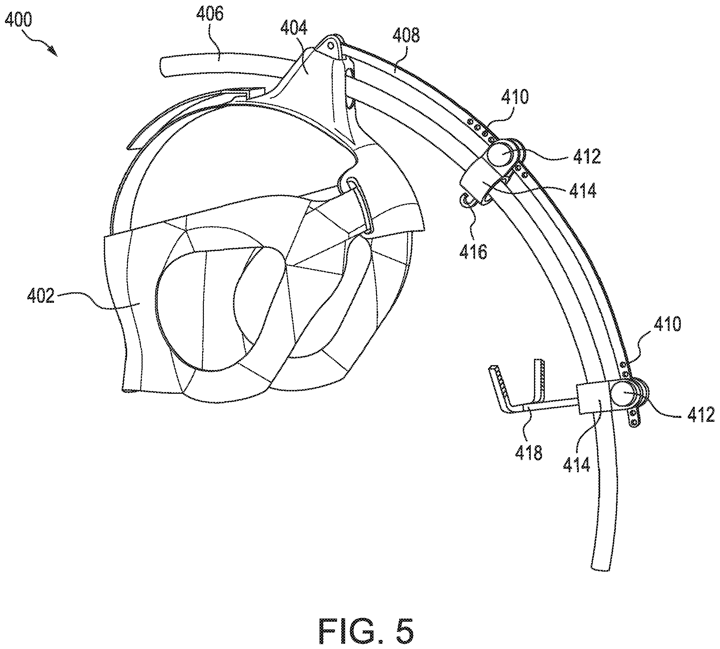

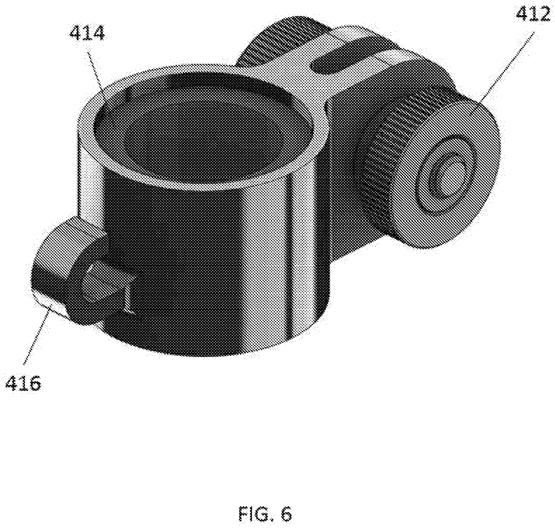

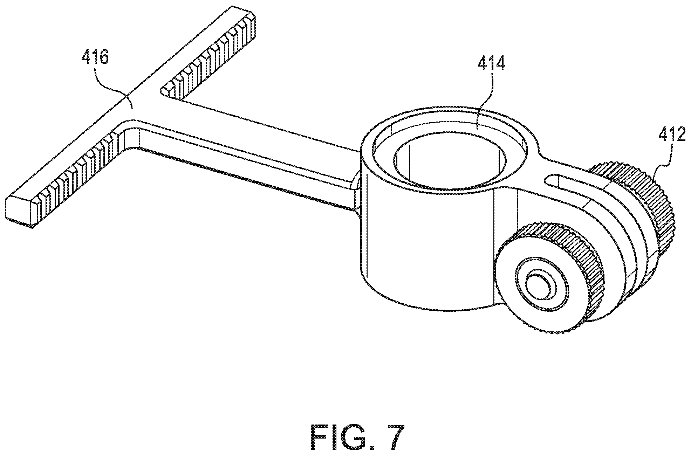

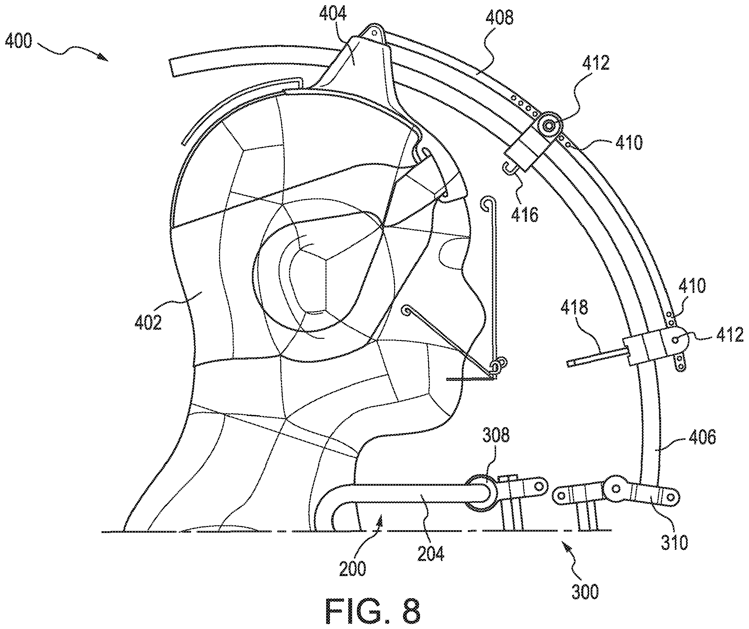

Referring now to Figure 5, headpiece **400** is described in detail. Headpiece **400** comprises head strap **402** attached to rail guide **404.** Head strap **402** is a flexible piece that is configured to wrap around a patient's head and holds rail guide **404** securely to the top of the patient's head. Rail guide **404** has a lumen sized to fit rail **406** and is connected to linkage bar **408.** In some embodiments, rail guide **404** comprises a low friction bearing within its lumen. Rail **406** and linkage bar **408** are aligned in parallel and share the same curvature. In some embodiments, the matching curvature can be described as a circular arc having an angle between about 90° and 140°. Rail **406** has a lower end that is attachable to headpiece attachment **310** of cantilever support **300.** Linkage bar **408** comprises a plurality of holes **410** along its curvature, each hole **410** being sized to fit a pin or screw from a lock **412.** Each lock **412** comprises a low friction bearing **414** that is slidable along rail **406.** In some embodiments, headpiece **400** comprises at least a first lock **412** having a hook attachment **416** and a second lock **412** having a linear gear bar attachment **418.** Hook attachment **416** (shown in greater detail in Figure 6) provides a point of attachment for a string, wire, or elastic band, and can include a simple hook, a closed loop, or a spring-loaded gate (such as a carabiner design). Linear gear bar attachment **418** (shown in greater detail in Figure 7) comprises one or more toothed bars to provide a variable point of attachment for a string, wire, or elastic band. Hook attachment **416** and linear gear bar attachment **418** are each connectable to a maxillary protraction device. The negative forces exerted on a maxillary protraction device can thereby be tuned by adjusting the position of each lock **412** with each hole **410** along the curvature of linkage bar **408.** For example, in one embodiment depicted in Figure 8, hook attachment **416** can be positioned between about 30° and 90° relative to a transverse plane, and linear gear bar attachment **418** can be positioned between about -35° and 60° relative to a transverse plane.

Combining body frame **200,** cantilever support **300,** and headpiece **400,** protraction device **100** is capable of comfortably applying protractionary forces to a patient without loading sensitive head and neck regions while permitting substantial freedom of movement in the patient's head. Locking each component on cantilever support **300** enables cantilever support **300** to be laterally slidable on body frame **200** as a single rigid unit by virtue of the slidable connection between its upper low friction bearing **308** to upper rail **204** and its lower low friction bearing **308** to lower rail **206.** With respect to headpiece **400,** head strap **402,** rail guide **404,** and each of the locks **412** can be locked in place relative to each other by way of linkage bar **408,** and the locked assembly is configured to be freely slidable along rail **406.** Device **100** thereby enables a patient to perform a shaking gesture along the curvature of upper rail **204** and lower rail **206** in the transverse plane to rotate the head left and right and a nodding gesture along the curvature of rail **406** in the median plane between flexion, extension, and hyper-extension of the neck. In some embodiments, headpiece attachment **310** further comprises a rotatable joint to permit a head tilting gesture in the coronal plane (i.e., lateral flexion). In some embodiments, one or more of clamps **306** can include a tension spring, or rotatable joints at headpiece attachment **310** and rail guide **404** can permit additional anterior and posterior head movement in the median plane (i.e. a pecking motion).

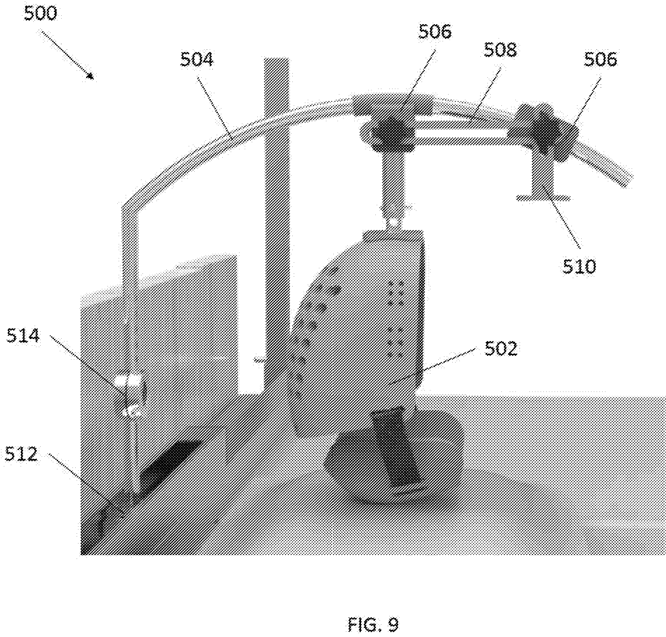

Referring now to Figure 9, another protraction device **500** is depicted. Protraction device **500** employs similar components as device **100** that can be anchored to a bed, gurney, or floor stand. Device **500** comprises head brace **502** attached to a curved rail **504** by a first lock **506.** Device **500** comprises one or more additional locks **506,** wherein each lock **506** is lockable relative to each other to linkage bar **508.** Locks **506** can each comprise a maxillary device attachment **510** to support loads for maxillary protraction. Rail **504** can be immobilized by anchor **512** to a headboard, floor stand, wall, or any suitable rigid structure. Rail **504** further comprises rotating hinge **514** to permit a patient to change sleeping positions while maintaining protractionary forces.

Additional research has identified optional modifications to the innovative cantilevered protraction device described above. A number of improvements have been developed that will enhance the functionality and durability of the device and improve the experience for the user.

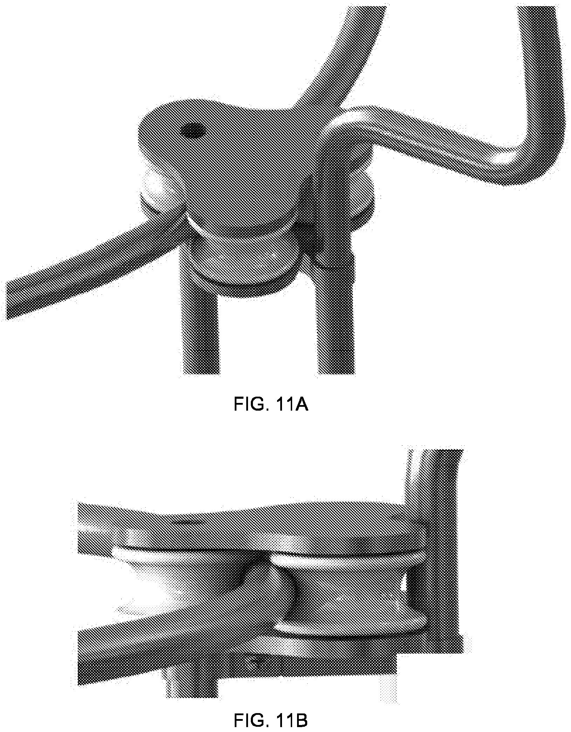

The lateral movement of the cantilever support along the body frame may be improved by the use of roller bearings. Figure 11A illustrates a perspective view of a cantilevered protraction device with a roller bearing. Figure 11B illustrates a side view of a cantilevered protraction device with a roller bearing. Roller bearings have a lower coefficient of friction than sliding bearings (such as the slider bearings shown in FIG. 4 as element **308** and in FIG. 5 as element **414**), which allows for more fluid motion when moment loads are applied.

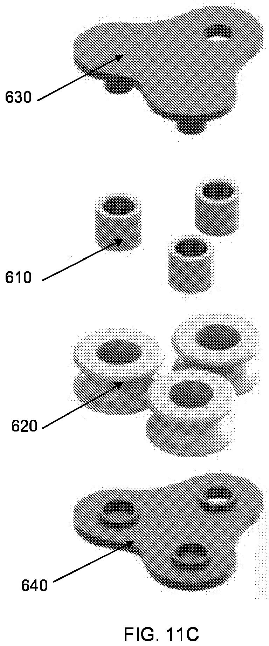

Figure 11C illustrates an exploded view of a roller bearing. The roller bearing includes one or more needle bearings **610** surrounded by an equal number of rollers **620** coupled to an upper plate **630** and a lower plate **640.** The components of the roller bearing may be coupled together using any suitable types of fasteners, such as screws or bolts. The upper and lower plates may be formed from any rigid, durable material such as metals, plastics or ceramics. Preferably, the upper and lower plates are made of plastic or aluminum. The rollers may be formed from any rigid, durable material such as metals, plastics or ceramics. Preferably, the rollers are made from plastic, aluminum or brass. The roller bearing may optionally include a low-friction substance between the rollers and the upper and lower plates to facilitate the motion of the rollers. The low-friction substance may be any material with a low coefficient of friction. Examples of suitable low-friction substances include polytetrafluoroethylene (PTFE or TEFLON^{®}), ultra high molecular weight polyethylene (UHMWPE), polyimide, polyether ether ketone (PEEK), polyphenylene sulfide (PPS), nylon, polyoxymethylene (POM or acetal), polyesters, acrylonitrile butadiene styrene (ABS), polycarbonate (PC) or polycarbonate/ABS (PC/ABS).

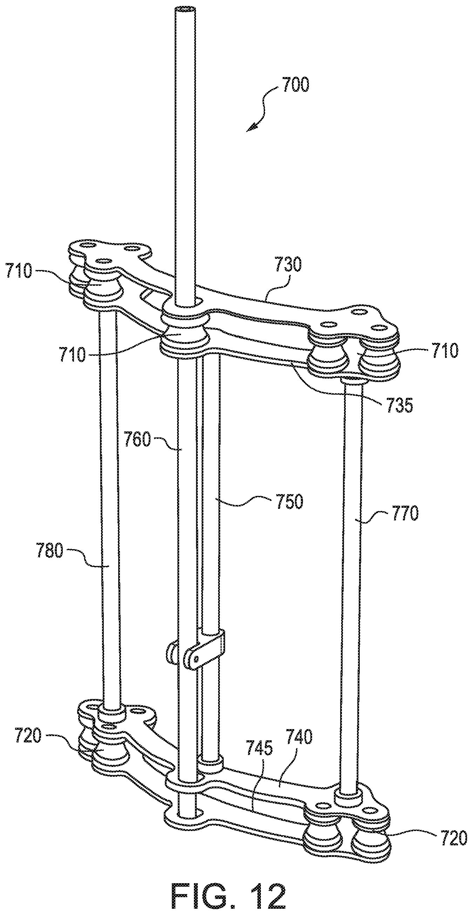

The lateral movement of the cantilever support along the body frame may also be improved by the use of multiple bearings. Preferably, the cantilevered protraction device includes a plurality of bearings on the upper and lower rails of the body frame. Figure 12 illustrates a cantilever support with multiple bearings. The cantilever support **700** includes a plurality of upper bearings **710** and a plurality of lower bearings **720,** which engage with the upper and lower rails of a body frame (not shown), respectively. The upper bearings are coupled together by a first upper bearing plate **730** and a second upper bearing plate **735.** The lower bearings are coupled together by a first lower bearing plate **740** and a second lower bearing plate **745.** The upper bearing plates and the lower bearing plates are coupled to a first shaft **750,** a second shaft **760,** a third shaft **770** and a fourth shaft **780.** The use of multiple bearings helps to distribute the load across a wider distance, which reduces the moment load on each individual bearing. The use of multiple bearings is especially helpful for patients receiving larger protractionary forces for therapy, such as mature patients.

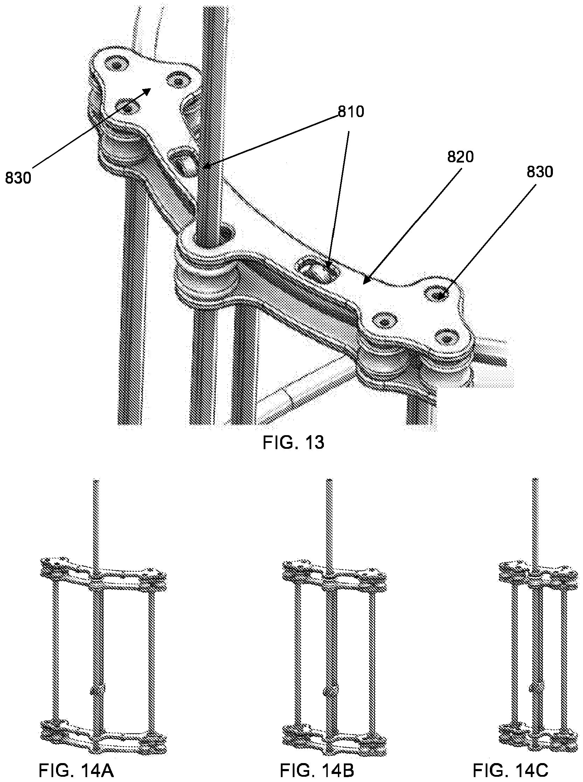

The cantilever support may optionally include additional rolling members to facilitate the lateral movement of the cantilever support along the body frame. Figure 13 illustrates a cantilever support with vertical rollers. Vertical rollers **810** may be coupled to a bearing plate **820** between roller bearings **830.** The vertical rollers may be coupled to any of the bearing plates. Additional rolling members may be used to smooth the lateral motion of the cantilever support when exposed to vertical loads.

In addition to securing the multiple bearings, the upper and lower bearing plates shown in Figures 12 and 13 improve the stability of the cantilever protraction device. Using a single plate to couple multiple bearings ensures that the roller bearings stay properly aligned with each other and with the rails of the body frame. In addition, the upper and lower bearing plates ensure that the first shaft and the second shaft maintain a proper vertical alignment. Maintaining vertical alignment of the first shaft and the second shaft is particularly important since these components may impair the lateral movement of the roller bearings if they become misaligned.

The use of multiple bearings allows for various geometries of the bearings and upper and lower plates. The separation angle of the bearings relative to the first shaft and the second shaft may be varied to provide a desired stability and movement profile. A wider separation angle spreads the moment loads and improves bearing performance. However, a wider angle will reduce the range of motion of the user's neck. A pair of roller bearings may have a separation angle of 15-90°. Preferably, a pair of roller bearings has a separation angle of 30-60°. More preferably, a pair of roller bearings has a separation angle of 45° or 60°. Figure 14A illustrates a cantilever support with multiple bearings having a separation angle of 60°. Figure 14B illustrates a cantilever support with multiple bearings having a separation angle of 45°. Figure 14C illustrates a cantilever support with multiple bearings having a separation angle of 30°.

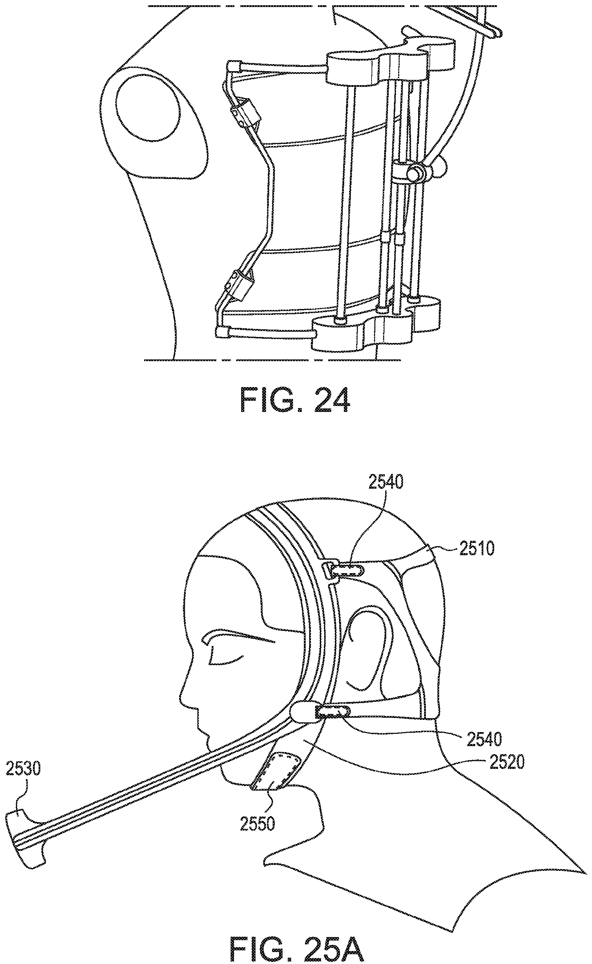

The bearings may optionally be concealed in a housing. Figure 24 illustrates a partial view of a cantilever protraction device with concealed bearings. Concealing the bearings protects the bearings from material that could impact their functioning, such as dirt or food, and prevents objects from being caught in the bearings, such as clothing or a patient's hair.

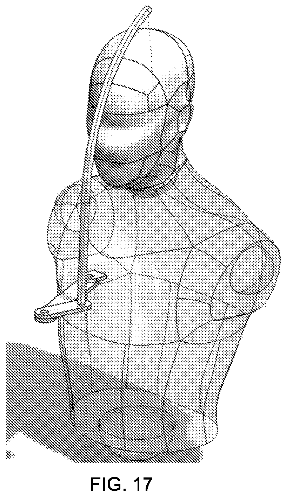

In an alternative configuration, a rotational hinge may be used to provide lateral movement of the cantilever support about the body frame. Figure 17 illustrates a cantilever support coupled to a rotational hinge, which may be coupled to a body harness (not shown).

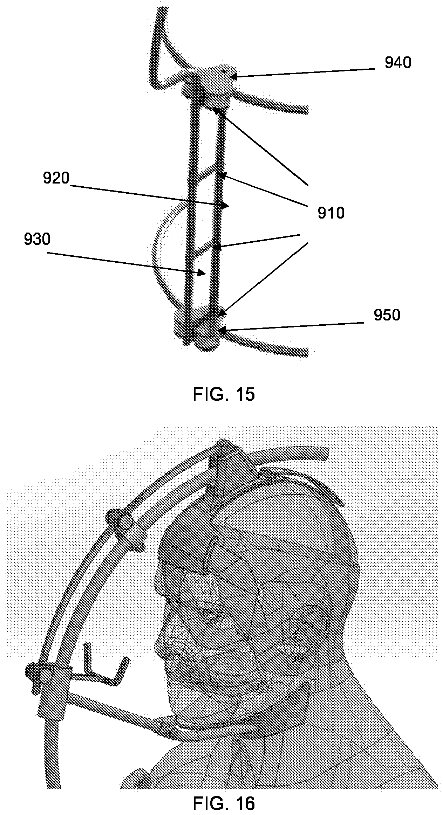

The stability of the cantilever protraction device may be increased by including support bars between the first shaft and the second shaft of the cantilever support. Figure 15 illustrates a cantilever support with support bars. A plurality of support bars **910** are coupled to a first shaft **920** and a second shaft **930.** The support bars are located between a first bearing **940** and a second bearing **950.** The support bars offer additional support beyond that provided by the clamps that couple the first shaft to the second shaft (see Figure 4, element **306**). Alternatively, the clamps may be replaced by support bars. The use of multiple support bars further improves the stability of the cantilever protraction device by reducing the moments applied to the bearings.

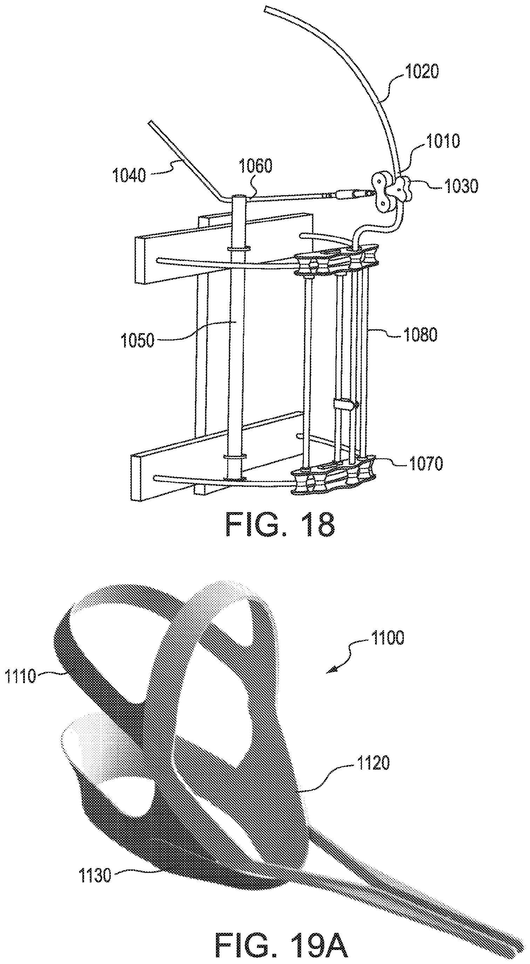

The comfort of the cantilever protraction device may be improved by including components that enhance the ability of a user to tilt, nod and rotate his or her head while wearing the device. Figure 18 illustrates a cantilever protraction device coupled to a moveable anchorage. The cantilever protraction device includes a force applicator **1010** coupled to a headpiece rail **1020** by a roller bearing **1030.** The headpiece rail is coupled to a cantilever support **1070** including a shaft **1080.** The headpiece rail and the shaft of the cantilever support may be monolithic. The force applicator is coupled to a therapeutic appliance (not shown) by a protraction wire **1040.** The protraction wire is coupled to a rotating post **1050** by a hinge **1060,** which represent the head and neck of a patient. The patient may tilt, nod and rotate his or her head while wearing the device and still receive a constant protractionary force during movement. The rotating post and hinge demonstrate that it is feasible to have the patient move the force applicator and the cantilever support by moving the protraction wire with his or her head.

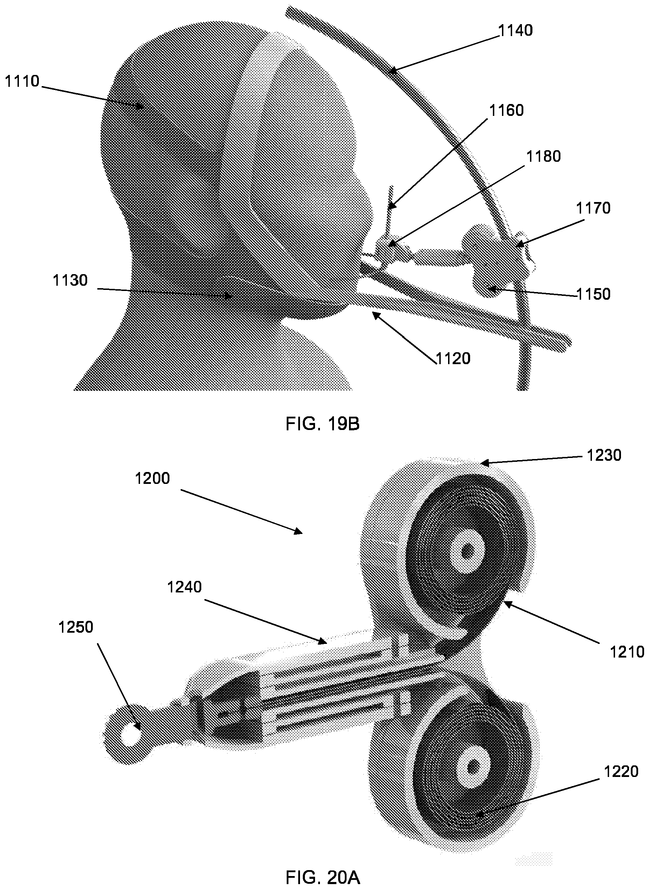

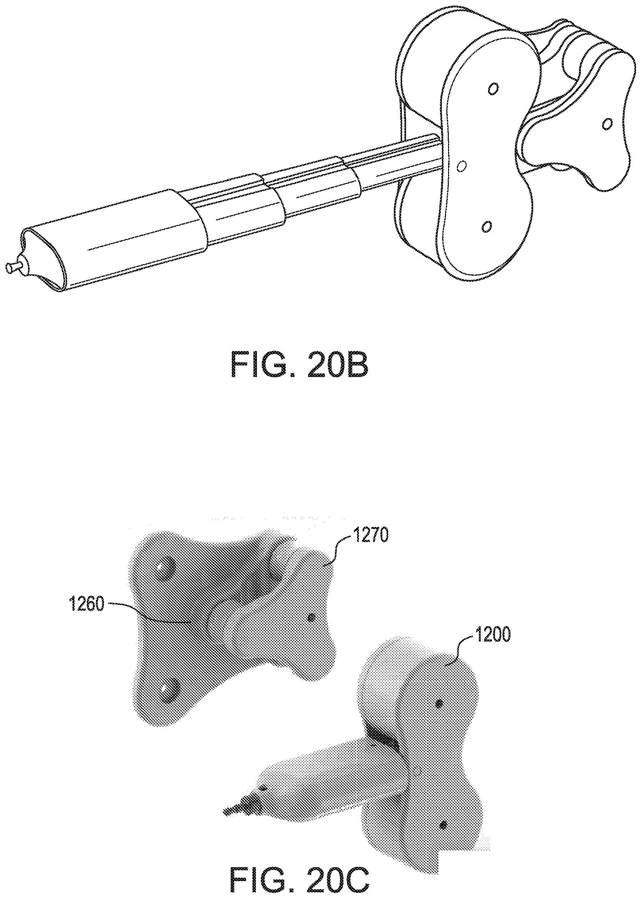

The force applicator preferably provides a constant force throughout the range of motion when the user tilts, nods or rotates his or her head while wearing the device. A preferred force applicator is a constant-force spring. Figure 20A illustrates a sectioned view of a force applicator including a constant-force spring. The force applicator **1200** includes a first constant-force spring **1210** and a second constant-force spring **1220** within a housing **1230.** The housing includes a telescoping sleeve **1240** that protects the spring when it is extended. The force applicator includes an attachment point **1250** for coupling the force applicator to a therapeutic appliance (not shown). Figure 20B illustrates a force applicator including a constant-force spring in an extended position. The force applicator preferably includes hard stops (not shown) to prevent overextension of the constant-force spring and exposure of the edges of the spring.

The force applicator may be removably coupled to a mount that is in turn coupled to a protraction device. Figure 20C illustrates a force applicator **1200** and a mount **1260** for coupling the force applicator to a protraction device (not shown). The force applicator may be removably coupled to the mount by a mechanical fastener, such as a screw or spring mechanism, or by non-mechanical means, such as magnets or friction (press fit or snap-fit). The mount includes a roller bearing **1270** that may be coupled to a protraction device (see FIG. 18 and FIG. 19B).

Force applicators may provide different amounts of force by varying the width, thickness and/or diameter of the constant-force springs within the force applicator. The force applicator may be configured to provide any suitable therapeutic force. Preferably, the force applicator provides 0.1 - 10 kg of force.

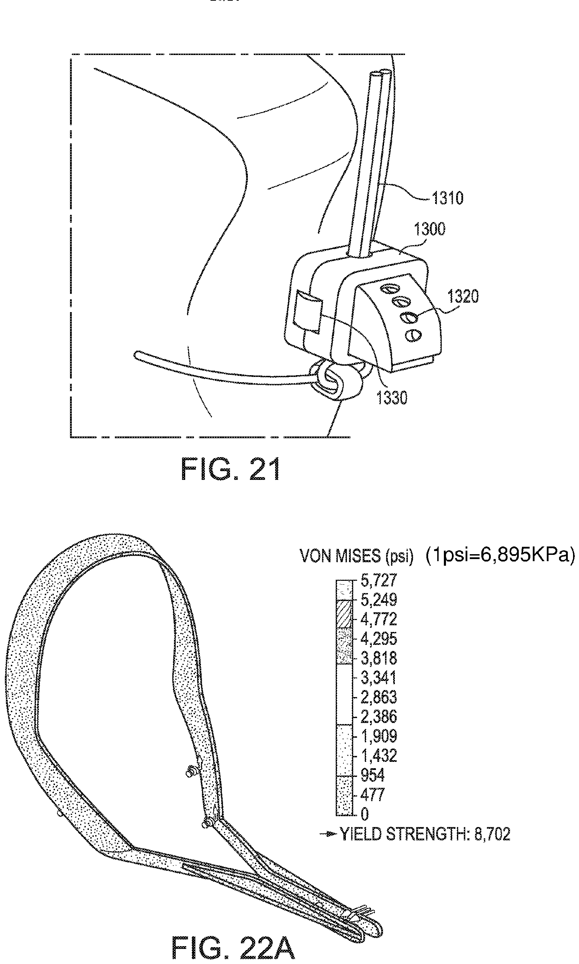

The force applicator may be coupled to a therapeutic appliance through an adjustable anchorage device. Figure 21 illustrates an adjustable anchorage device **1300** coupled to a protraction wire **1310.** The adjustable anchorage device includes a plurality of holes **1320** for receiving a force applicator (see FIG. 19B). The holes may provide a range of angles for the force applicator, such as 0°- 45°, including 5°, 10°, 15°, 20°, 25°, 30°, 35° and 40°. The adjustable anchorage device may be removably coupled to the protraction wire by actuating a release mechanism **1330.** The adjustable anchorage device may be removably coupled to the protraction wire by a mechanical fastener, such as a screw or spring mechanism, or by non-mechanical means, such as magnets or friction (press fit or snap-fit).

The comfort of the cantilever protraction device may also be improved by customizing the body frame (see Figure 2). One way to adjust the body frame is by making the strap pads on the body frame removable. For example, the strap pads may be removably coupled to the body frame using fasteners such as snaps, hook and loop fasteners or buckles. The use of removable strap pads allows the fit of the device to be customized to each specific user. For example, strap pads with differing levels of padding or differing levels of flexibility may be selected to provide a desired fit. The strap pads are preferably machine washable. Another way to adjust the body frame is by varying the geometry of the body frame rails. For example, the body frame rails may be straight, angled, curved, or some combination of these geometries. The customization of the body frame will promote a comfortable fit and promote user compliance.

Reducing the weight of the cantilever protraction device significantly improves the comfort for the user. The cantilever protraction device preferably includes materials that have high strength, high rigidity and low weight. Examples of suitable materials include aluminum, titanium, and carbon fiber.

The weight of the cantilever protraction device may be reduced by the use of hollow rather than solid components. For example, the rails of the body frame may be hollow tubes. Preferably, the hollow tubes are circular in cross section. The use of tubular rails in the body frame facilitates the movement of the bearings along the rails. The use of hollow components also reduces the cost of manufacturing the cantilever protraction device. Hollow tubes are readily available, easy to manufacture and inexpensive. Hollow aluminum tubes are a particularly preferred material for use in the body frame.

The comfort of the cantilever protraction device may be significantly improved by modifying the headpiece of the device. Figure 19A illustrates a headpiece for a cantilever protraction device. The headpiece **1100** includes straps **1110** coupled to a harness **1120.** The headpiece may optionally include a mandible strap **1130.** The straps and the harness may optionally include pads on the side that contacts the user's head for comfort. The configuration of the mandible strap may be varied to provide a customized fit for the user. For example, the mandible strap may be configured to pass over or under the user's chin. In addition, the mandible strap may be configured to secure to both sides of a user's head. The harness may be formed from any rigid, durable material such as metals, plastics or ceramics. Preferably, the harness is made of plastic.

Figure 19B illustrates a headpiece for a cantilever protraction device as worn by a user. The straps **1110** are secured above and below the user's ears. The mandible strap **1130** passes under the user's chin. The harness **1120** is coupled to a headpiece rail **1140.** A force applicator **1150** is coupled to a protraction wire **1160** and is coupled to the headpiece rail by a roller bearing **1170.** The protraction wire is coupled to a therapeutic appliance (not shown) by an adjustable anchorage device **1180.** The harness is preferably coupled to the headpiece rail close to the protraction wire to provide optimum support. Unlike conventional protraction devices, the harness does not obstruct the facial area of the user. Having an unobstructed facial area will improve comfort and facilitate use by users who wear eyeglasses or sunglasses.

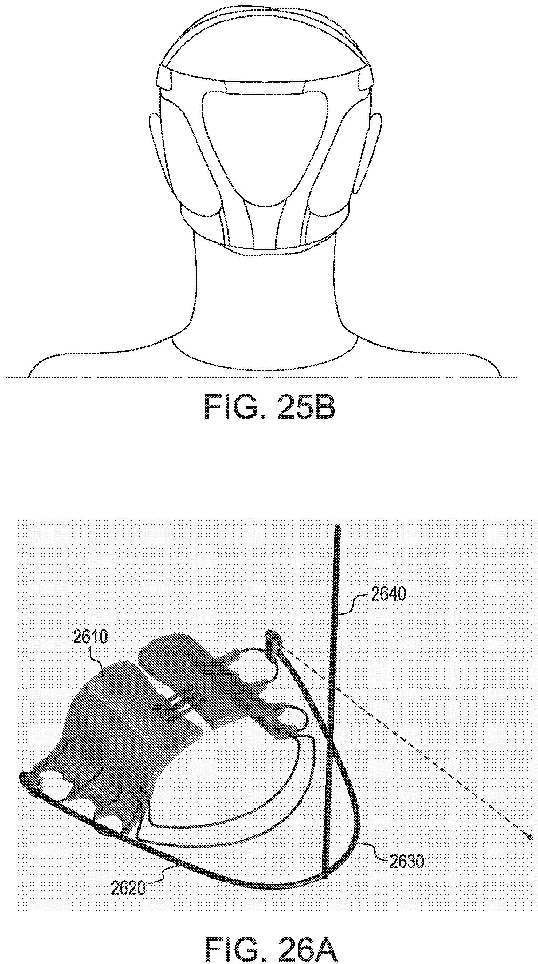

Figure 25A illustrates a side view of an alternative design of a headpiece for a cantilever protraction device as worn by a user. Figure 25B illustrates a back view of the headpiece. The headpiece includes a first strap **2510** that encircles the back and sides of the user's head and a second strap **2520** that passes under the user's mandible and is removably coupled to the user's head with a mandible fastener **2550.** A harness **2530** is coupled to the second strap and is removably coupled to the first strap by a plurality of harness fasteners **2540** and to a protraction device (not shown). The mandible and harness fasteners are adjustable to provide a customized fit. The fasteners may be any suitable type of removable fastener, such as a hook and loop (VELCRO^{®}) fastener, a magnetic fastener or a snap fastener. In addition to improving the comfort of the headpiece, the fasteners may also simplify the process of attaching and removing the headpiece from the user's head. For example, a user may customize the fit of the first strap and secure the harness to the first strap with hook and loop harness fasteners. The headpiece may then be attached to or removed from the user's head by fastening or unfastening a magnetic mandible fastener near the user's chin or cheek without the need to readjust the harness fasteners.

The headpiece may optionally include a neck support. Figure 16 illustrates a cantilever protraction device with a neck support. The neck support may be used to apply forces on the mandible or support the neck during therapy.

The updated headpiece designs shown in Figures 19A and 19B offers significant advantages over the earlier headpiece design shown in Figures 1-9. Components such as the linkage bar (Figure 5, element **408**) and the rail guide (Figure 5, element **404**) may be eliminated, which reduces cost and the weight of the device. The use of a rigid force applicator and roller bearings in the updated headpiece design also improves the functionality of the device. The earlier headpiece design required the user to use his or her head to direct the movement of the cantilever support along the body frame and the movement of the headpiece components along the headpiece rail. This head-based movement resulted in undesired forces on the user's head and neck areas. By contrast, in the updated headpiece design the rigid force applicator assists in the movement of the device components, particularly when nodding. This allows the updated headpiece to be used to maintain the user's head in the proper position and assist with lateral motion of the cantilever support along the body frame, reducing the undesired forces on the user's head and neck areas.

A key feature of the cantilever protraction device is that it is universally compatible with any orthodontic and craniomaxillofacial (CMF) devices. The cantilever protraction device may be coupled to a therapeutic appliance anchored to the teeth, bone, such as the upper jaw or lower jaw, or soft tissue of a patient. For example, the cantilever protraction device may be coupled to the maxillary protraction devices described in WO 2019/018249 and WO 2019/104255. Similarly, any type of force applicator may be coupled to the cantilever protraction. For example, the force applicator may include springs, constant-force springs, elastics and wires.

Another key feature of the cantilever protraction device is that it does not apply any forces or loads to the head of the user. As shown in the figures and as described above, a headpiece is coupled to a cantilever support, but the cantilever support does not impart any forces to the user's head through the headpiece. For example, Figure 5 illustrates that the rail **406** passes through an opening in the rail guide **404,** but the rail does not rest on the rail guide or transmit any forces onto the head of the user. In the embodiment shown in Figure 5, the rail and the rail guide only are in contact when the user moves his or her head laterally, which results in lateral movement of the cantilever support about the body frame.

A means for movably coupling a cantilever support to a body frame may be, for example, a slider bearing, a roller bearing, a vertical roller or a rotational hinge. A means for coupling the head of a patient to a cantilever support may be, for example, a headpiece, a head strap, a harness or a harness including a head strap and/or a mandible strap. A means for applying a protractionary force to the craniofacial complex of a patient may be, for example, a spring, a constant-force spring, elastics or a wire.

### Protraction Wire Customization

A preferred application of the cantilever protraction device is use with a skeletal anchorage system to apply an extra-oral force, such as a protraction force, to an intra-oral skeletal anchorage. As described in WO 2019/018249 and WO 2019/104255, it is possible to apply non-rotational forward and forward and upward forces directly to the maxilla of a patient. This is preferable to conventional devices that involve attaching elastics to an intra-oral anchorage, which results in an unnatural downward pull and/or rotational pull on the maxillary complex.

The extra-oral forces may be applied to the intra-oral skeletal anchorage using an orthodontic appliance that can be described as a modified orthodontic facebow, wire or protraction wire. The orthodontic appliance may be removably coupled to the skeletal anchorage by the patient or his or her caregiver. Due to the natural variation in each patient's oral and bone geometry, the positioning of the skeletal anchorage device and the configuration of the protraction wire must be customized for each patient.

A method of customizing a protraction wire includes determining a patient's oral and/or bone geometry; and adjusting the protraction wire. The protraction wire includes an intra-oral portion that is located inside the mouth of the patient and an extra-oral portion that is located outside of the mouth of the patient. The intra-oral portion of the protraction wire may be adjusted to match the patient's oral and/or bone geometry. For example, the protraction wire may be adjusted to minimize interference and/or friction with the lips, cheeks, teeth, gingiva, and the tongue to minimize disruption of biological functions. The extra-oral portion of the protraction wire may be adjusted to provide a specific therapeutic force application. For example, if the patient requires lateral force application to the skeletal anchorage point, the width of the protraction wire may be increased to impart a desired lateral force. Adjusting the protraction wire may involve adjusting the intra-oral portion of the protraction wire, the extra-oral portion of the protraction wire or both the intra-oral and the extra-oral portions of the protraction wire. Any adjustments to the protraction wire must maintain optimal coupling with a skeletal anchorage device.

The protraction wire may be composed of any substance that is sufficiently rigid to transmit therapeutic forces to the patient and that is sufficiently malleable to be adjusted to fit the patient's specific oral and/or bone geometry. For example, the protraction wire may be composed of spring temper wire or annealed wire. A preferred protraction wire material is 17-7 PH stainless steel.

A patient's oral and/or bone geometry may be determined using any suitable means for measuring and recording the details of the patient's oral and/or bone geometry. Examples of measuring and recording techniques include obtaining an impression, such as by dental casting or molding, and obtaining a digital measurement, such as by a digital scan or cone beam computed tomography (CBCT). A physical or digital model of the patient's oral and/or bone geometry may be created from the recorded measurements.

The protraction wire may be adjusted manually. A physical model of the patient's oral and/or bone geometry may be coupled to a dental rig. A protraction wire may then be adjusted by hand or using tools to match the model of the patient's oral and/or bone geometry to provide a specific therapeutic force application.

The protraction wire also may be adjusted digitally. A three-dimensional (3D) model of the patient's oral and/or bone geometry may be created from a digital measurement using a 3D software module. A digital model of a protraction wire may be adjusted to match the digital model of the patient's oral and/or bone geometry to provide a specific therapeutic force application. The digital model of the protraction wire may be submitted to a wire former to manufacture the customized protraction wire, or may be used to produce a customized protraction wire using a desktop 3D wire former. Machine learning may optionally be used to accelerate the digital adjustment process.

Adjusting the protraction wire may optionally involve the input of a medical professional experienced with orthodontic and craniomaxillofacial (CMF) therapy. For example, the medical professional may be a doctor, a dentist, an orthodontist or an assistant/technician working under the supervision of the medical professional. The medical professional may aid in determining the appropriate therapeutic force vectors to provide a desired therapeutic outcome.

The protraction wire may optionally be treated after customization. For example, a customized protraction wire may be heat treated to reach a desired hardness and yield strength. The protraction wire may be treated by the person who customizes the wire. Alternatively, the protraction wire may be treated at a separate facility in a different physical location from the customization.

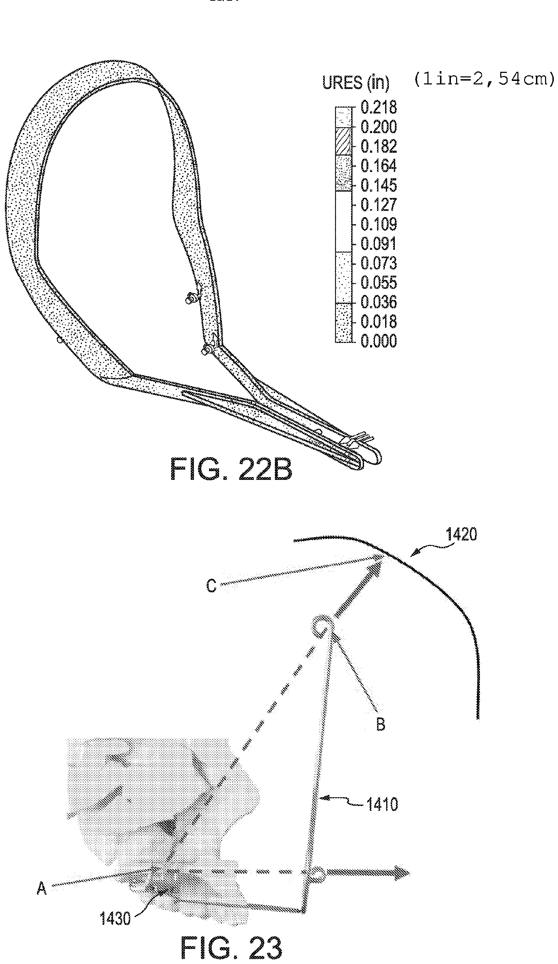

Figure 23 illustrates a protraction wire **1410** coupled to a protraction device **1420** and to a skeletal anchorage **1430.** A non-rotational forward and/or forward/upward force vector may be applied to the patient's maxilla along a desired force vector of 0°-75° by aligning the skeletal anchorage (Point A), the protraction wire (Point B) and the protraction device (Point C). The appropriate location of the skeletal anchorage, the protraction wire and the protraction device is unique to each patient and is determined by the patient's oral and/or bone geometry and therapeutic force needed. The protraction wire may be customized as discussed above. The protraction wire and the protraction device may optionally be configured to provide adjustable force vectors. For example, the protraction wire may be coupled to the protraction device by an adjustable force applicator and an adjustable anchorage device (see FIG. 19B). Similarly, the protraction wire may include multiple attachment points or notches to provide variable force vectors.

The several components of the present invention described above can be constructed using any suitable method known in the art. The method of making may vary depending on the materials used. For example, components substantially comprising a metal may be milled from a larger block of metal or may be cast from molten metal. Likewise, components substantially comprising a plastic or polymer may be milled from a larger block, cast, or injection molded. In some embodiments, the devices may be made using 3D printing or other additive manufacturing techniques commonly used in the art.

### EXAMPLES

Some elements of the invention are further described in detail by reference to the following experimental examples. These examples are provided for purposes of illustration only, and are not intended to be limiting unless otherwise specified.

Without further description, it is believed that one of ordinary skill in the art can, using the preceding description and the following illustrative examples, make and utilize the compounds of the present invention. The following working examples therefore, specifically point out exemplary embodiments and are not to be construed as limiting in any way the remainder of the disclosure.

### Example 1: Low friction bearing prototyping

A low friction bearing was attached to a leveled aluminum tube and a known weight was hung from the bearing to apply a normal force. A container was attached to the same bearing and the wire was run perpendicularly to and hung from a pulley wheel. Weights were added to the container to determine the amount of lateral force needed to move the bearing and overcome the normal force applied by the known weight.

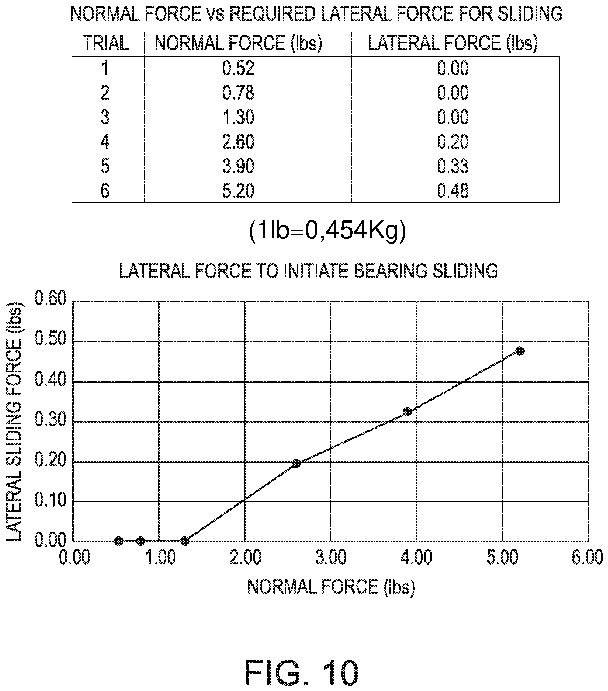

Results are shown in Figure 10. The lateral force required to initiate sliding in the bearing is more than an order of magnitude less than the applied therapeutic loads. For example, the nominal case of 2.2 lbs forward force plus 2.2 Ibs upward force has a resultant force of (2.2² + 2.2²)^{0.5} = 3.1 lbs. This would require 0.26 Ibs of a head turning force to initiate sliding. An extreme case of 4.4 lbs forward force plus 4.4 Ibs upward force has a resultant force of (4.4² = 4.4²)^{0.5} = 6.2 lbs. This would require 0.52 lbs head turning force. The initiation force is the peak force needed to start motion from a dead stop. Once motion is started, the friction (and subsequent forces) can drop by as much as 50% (1lb=0,454Kg).

### Example 2: Finite element analysis (FEA) of headpiece harness

A finite element analysis (FEA) was performed on the headpiece harness shown in Figures 19A and 19B. The FEA modeled the stresses and displacements on the harness when subjected to up to 2 lbs of pushing force. Figure 22A illustrates the finite element analysis of the von Mises stresses of the harness as measured in pounds per square inch (psi). Figure 22B illustrates the finite element analysis of the URES displacement of the harness as measured in inches. The harness exhibited acceptable stresses and displacement with up to 2 lbs of pushing force (1lb=0,454Kg).

### Example 3: Use of cantilever protraction device with acrylic appliance

The BIOBLOC^{™} acrylic appliance is a commercially available therapeutic appliance used to generate craniofacial skeletal corrections to treat craniofacial dystrophy. The BIOBLOC^{™} appliance is suitable for adolescent patients who have open sutural system/synchondroses (growth centers) in their maxillofacial complex, which prevents the need for skeletal anchorage. The conventional use of the BIOBLOC^{™} appliance involves coupling the appliance to a protraction wire that extends towards the patient's ears. This design is uncomfortable for patients, particularly when the appliance is worn overnight since it impacts the ability of patients to sleep comfortably on their sides. In addition, the use of a protraction wire that extends towards the ears results in a force vector that extends in a different direction than the other therapeutic forces provided by the protraction device.

Figure 26A illustrates a perspective view of a therapeutic appliance **2610** coupled to a trans-oral member **2620.** The trans-oral member includes a curved member **2630** and an extra-oral vertical member **2640.** The therapeutic appliance is adapted to be coupled to at least one member selected from the group consisting of the teeth, bone, and soft tissue of the patient. Each end of the curved member is coupled to opposing ends of the therapeutic appliance. The extra-oral vertical member extends vertically from the plane of the curved member, and may optionally extend perpendicularly or substantially perpendicularly from the curved member. The trans-oral member may be coupled to a protraction device (not shown), such as the cantilever protraction device described herein.

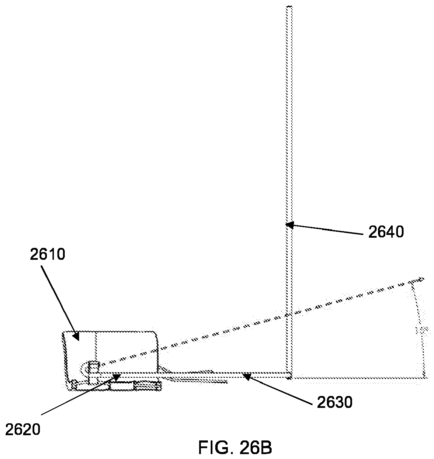

The dashed line in Figure 26A represents the direction of the force vector. Figure 26B illustrates a side view of the therapeutic appliance coupled to the trans-oral member. The dashed line in Figure 26B indicates that the force vector extends at an angle of 15° relative to the trans-oral member. Importantly, the force vector extends in the same direction as the trans-oral member and any protractionary forces imparted by an extraoral force applicator (not shown). Preferably, the protractionary forces are only applied through the extra-oral vertical member.

It should be understood that the BIOBLOC^{™} appliance is exemplary of any tooth-anchored appliance. Applying protraction forces to any tooth-anchored appliance provides the same result: the protraction forces are transmitted to the tooth roots and from there to the palatal vault. The trans-oral member may be attached to any type of tooth-anchored appliance, including directly to molar attachments without any palatal components.

The scope of the invention is defined by the claims.

### REFERENCES

1. U.S. Patent No. 8,640,710.
2. U.S. Patent No. 10,166,089.
3. U.S. Patent Publication No. 2018/0028282.
4. Moon, W., "Class III treatment by combining facemask (FM) and maxillary skeletal expander (MSE)", Seminars in Orthodontics, Vol 24, No. 1, pp. 95-107 (2018).
5. International Patent Publication No. WO 2019/018249.
6. International Patent Publication No. WO 2019/104255.

## Claims

1. A protraction device (100), comprising:
a body frame (200), adapted to be anchored to the chest and abdomen of a patient,
a cantilever support (300), including
a first shaft (302), coupled to the body frame, and
a second shaft (304), coupled to the first shaft,
and
a headpiece (400) coupled to the second shaft and adapted to be attached to the head of the patient,
wherein the second shaft extends in front of the face of the patient when the protraction device is worn by said patient,
**characterized in that** the body frame comprises
a top rail (204), and
a bottom rail (206), and
the cantilever support further comprises
a top bearing (308), coupled to the top rail, and
a bottom bearing (308), coupled to the bottom rail, and
the first shaft is coupled to the top bearing and the bottom bearing.

2. The protraction device of claim 1, wherein the top bearing and the bottom bearing comprise roller bearings.

3. The protraction device of claim 1, wherein the headpiece comprises
a harness (1120), and
a head strap (1110), coupled to the harness, and
the headpiece is coupled to the second shaft by the harness.

4. The protraction device of claim 2, wherein the cantilever support comprises at least two top bearings (710) and at least two bottom bearings (720),
the at least two top bearings are coupled by a first top bearing plate (730) and a second top bearing plate (735), and
the at least two bottom bearings are coupled by a first bottom bearing plate (740) and a second bottom bearing plate (745).

5. The protraction device of claim 4, wherein the first top bearing plate, the second top bearing plate, the first bottom bearing plate and the second bottom bearing plate are each monolithic.

6. The protraction device of claim 1, wherein the body frame further comprises a first side rail (207), coupled to one end of the top rail and the bottom rail,
a second side rail (207), coupled to the opposite end of the top rail and the bottom rail, and
a plurality of straps, coupled to the body frame.

7. The protraction device of claim 3, wherein the head strap further comprises a mandible strap (1130).

8. The protraction device of claim 1, further comprising a force applicator mount (1260), coupled to the second shaft by a bearing (1270).

9. The protraction device of claim 8, further comprising a force applicator (1200), removably coupled to the force applicator mount.

10. The protraction device of claim 9, further comprising a therapeutic appliance, coupled to the force applicator.

11. The protraction device of claim 10, wherein the therapeutic appliance is coupled to the force applicator by an adjustable anchorage device (1300).

12. The protraction device of claim 4, further comprising a low-friction substance between the roller bearings and the bearing plates.

13. The protraction device of claim 4, further comprising a vertical roller (810) coupled to at least one of the first top bearing plate, the first bottom bearing plate, the second top bearing plate and the second bottom bearing plate.

## Patentansprüche

1. Eine Oberkieferdauerzugvorrichtung (100), die Folgendes umfasst:
einen Körperrahmen (200), der an Brust und Bauch eines Patienten verankert werden kann, eine Auslegerhalterung (300), einschließlich
ein erster Schaft (302), der mit dem Körperrahmen verbunden ist, und
ein zweiter Schaft (304), der mit dem ersten Schaft des Patienten verbunden ist,
und
ein Kopfstück (400), das mit dem zweiten Schaft verbunden ist und am Kopf des Patienten angebracht werden kann,
wobei sich der zweite Schaft vor dem Gesicht des Patienten erstreckt, wenn die Oberkieferdauerzugvorrichtung vom Patienten getragen wird,
die **dadurch gekennzeichnet ist, dass** der Körperrahmen
eine obere Schiene (204) und
eine untere Schiene (206) umfasst, und
die Auslegerhalterung ferner
ein oberes Lager (308), das mit der oberen Schiene verbunden ist,
und ein unteres Lager (308), das mit der unteren Schiene verbunden ist, umfasst, und
der erste Schaft mit dem oberen Lager und dem unteren Lager verbunden ist.

2. Die Oberkieferdauerzugvorrichtung gemäß Forderung 1, wobei das obere Lager und das untere Lager Rollenlager umfassen.

3. Die Oberkieferdauerzugvorrichtung gemäß Forderung 1, wobei das Kopfstück
einen Gurt (1120) und
einen Kopfriemen (111O) umfasst, der mit dem Gurt verbunden ist, und
das Kopfstück mit dem zweiten Schaft durch den Gurt verbunden ist.

4. Die Oberkieferdauerzugvorrichtung gemäß Forderung 2, wobei die Auslegerhalterung mindestens zwei obere Lager (710) und mindestens zwei untere Lager (720) umfasst,
wobei die mindestens zwei oberen Lager durch eine erste obere Lagerplatte (730) und eine zweite obere Lagerplatte (735) gekoppelt sind, und
die mindestens zwei unteren Lager durch eine erste untere Lagerplatte (740) und eine zweite untere Lagerplatte (745) gekoppelt sind.

5. Die Oberkieferdauerzugvorrichtung gemäß Forderung 4, wobei die erste obere Lagerplatte, die zweite obere Lagerplatte, die erste untere Lagerplatte und die zweite untere Lagerplatte jeweils monolithisch sind.

6. Die Oberkieferdauerzugvorrichtung gemäß Forderung 1, wobei der Körperrahmen weiterhin eine erste Seitenschiene (207), die mit einem Ende der oberen Schiene und der unteren Schiene verbunden ist,
eine zweite Seitenschiene (207), die mit dem gegenüberliegenden Ende der oberen Schiene und der unteren Schiene verbunden ist,
und
eine Vielzahl von Gurten, die mit dem Körperrahmen verbunden sind, umfasst.

7. Die Oberkieferdauerzugvorrichtung gemäß Forderung 3, wobei der Kopfgurt außerdem einen Unterkiefergurt (1130) umfasst.

8. Die Oberkieferdauerzugvorrichtung gemäß Forderung 1 umfasst außerdem eine Kraft-Applikator-Halterung(1260), die über ein Lager (1270) mit dem zweiten Schaft verbunden ist.

9. Die Oberkieferdauerzugvorrichtung gemäß Forderung 8 umfasst außerdem einen Kraft-Applikator (1200), der abnehmbar mit der Kraft-Applikatorhalterung verbunden ist.

10. Die Oberkieferdauerzugvorrichtung gemäß Forderung 9 umfasst außerdem eine therapeutische Vorrichtung, die mit dem Kraft-Applikator verbunden ist.

11. Die Oberkieferdauerzugvorrichtung gemäß Forderung 10, wobei das therapeutische Gerät mit dem Kraft-Applikator durch eine einstellbare Verankerungsvorrichtung (1300) verbunden ist.

12. Die Oberkieferdauerzugvorrichtung gemäß Forderung 4 umfasst ferner eine reibungsarme Substanz zwischen den Rollenlagern und den Lagerplatten.

13. Die Oberkieferdauerzugvorrichtung gemäß Forderung 4 umfasst ferner eine vertikale Rolle (810), die mit mindestens einer der folgenden Platten gekoppelt ist: Die erste obere Lagerplatte, die erste untere Lagerplatte, die zweite obere Lagerplatte und die zweite untere Lagerplatte.

## Revendications

1. Dispositif de protraction (100), comprenant :
un cadre de corps (200), adapté pour être ancré à la poitrine et à l'abdomen d'un patient,
un support en porte-à-faux (300), incluant
une première gaine (302), couplée au cadre de corps, et
une deuxième gaine (304), couplée à la première gaine,
et
une pièce de tête (400) couplée à la deuxième gaine et adaptée pour être fixée à la tête du patient,
dans laquelle la seconde gaine s'étend devant le visage du patient lorsque celui-ci porte le dispositif de protraction,
**caractérisé par le fait que** le cadre du corps comprend
un rail supérieur (204), et
un rail inférieur (206), et
le support en porte-à-faux comprend en outre
un palier supérieur (308), couplé au rail supérieur, et
un palier inférieur (308), couplé au rail inférieur, et
la première gaine est couplée au palier supérieur et au palier inférieur.

2. Le dispositif de traction de la revendication 1, dans lequel le palier supérieur et le palier inférieur comprennent des paliers à rouleaux.

3. Le dispositif de traction de la revendication 1, dans lequel la pièce de tête comprend
un harnais (1120), et
une sangle de tête (1110), couplée au harnais, et
la pièce de tête est couplée à la deuxième gaine par le harnais.

4. Le dispositif de traction de la revendication 2, dans lequel le support en porte-à-faux comprend minimum deux paliers supérieurs (710) et minimum deux paliers inférieurs (720),
les minimum deux paliers supérieurs sont couplés par une première plaque de palier supérieur (730) et une seconde plaque de palier supérieur (735), et
les minimum deux paliers inférieurs sont couplés par une première plaque de palier inférieur (740) et une seconde plaque de palier inférieur (745).

5. Le dispositif de traction de la revendication 4, dans lequel la première plaque de palier supérieur, la seconde plaque de palier supérieur, la première plaque de palier inférieur et la seconde plaque de roulement inférieur sont toutes monolithiques.

6. Le dispositif de protraction de la revendication 1, dans lequel le cadre de corps comprend un premier rail latéral (207), couplé à une extrémité du rail supérieur et du rail inférieur,
un second rail latéral (207), couplé à l'autre extrémité du rail supérieur et du rail inférieur, et
plusieurs sangles, couplées au cadre de corps.

7. Le dispositif de protraction de la revendication 3, dans lequel la sangle de tête comprend en outre une sangle mandibulaire (1130).

8. Le dispositif de protraction de la revendication 1, comprenant en outre un support d'applicateur de force (1260), couplé à la seconde gaine par un palier (1270).

9. Le dispositif de protraction de la revendication 8, comprenant en outre un applicateur de force (1200), couplé de façon amovible au support d'applicateur de force.

10. Le dispositif de protraction de la revendication 9, comprenant en outre un appareil thérapeutique, couplé à l'applicateur de force.

11. Le dispositif de protraction de la revendication 10, dans lequel l'appareil thérapeutique est couplé à l'applicateur de force par un dispositif d'ancrage réglable (1300).

12. Le dispositif de protraction de la revendication 4, comprenant en outre une substance à faible friction entre les paliers à rouleaux et les plaques de palier.

13. Le dispositif de protraction de la revendication 4, comprenant en outre un rouleau vertical (810) couplé à au moins un parmi la première plaque de palier supérieur, la première plaque de palier inférieur, la seconde plaque de palier supérieur et la seconde plaque de palier inférieur.
